# EUROPÄISCHE PATENTSCHRIFT

(11) **EP 1 501 556 B1**
(45) Veröffentlichungstag und Bekanntmachung des Hinweises auf die Patenterteilung: **26.10.2005**
(21) Anmeldenummer: 03747414.5
(22) Anmeldetag: 24.04.2003
(51) Int. Cl.: A61L 2/22, E04H 1/12, A01K 1/015, B08B 3/00, B01F 3/04, B25J 21/00

(54) **VORRICHTUNG ZUR ERZEUGUNG EINES SCHAUMTEPPICHS, DAMIT AUSGESTATTETE HYGIENESCHLEUSE, BEHANDLUNGSVERFAHREN UND VERWENDUNG DER VORRICHTUNG**
DEVICE FOR THE PRODUCTION OF A CARPET OF FOAM, HYGIENE LOCK COMPRISING SAID DEVICE, METHOD OF TREATMENT, AND USE OF SAID DEVICE
DISPOSITIF DE PRODUCTION D'UN TAPIS MOUSSE, SAS SANITAIRE EQUIPE DE CE DISPOSITIF, PROCEDE DE TRAITEMENT ET UTILISATION DE CE DISPOSITIF

(30) Priorität: 03.05.2002 DE 10219780
(43) Veröffentlichungstag der Anmeldung: 02.02.2005
(73) Patentinhaber: ECOLAB INC., St. Paul, MN 55102-1390 (US)
(72) Erfinder: OUZOUNIS, Dimitrios, 47803 Krefeld (DE); DE ROQUEFEUIL, Hubert, 78490 Montfort Lamaury (FR)
(74) Vertreter: Sternagel, Fleischer, Godemeyer & Partner
(86) Internationale Anmeldenummer: PCT/EP2003/004254
(87) Internationale Veröffentlichungsnummer: WO 2003/092747

(56) Entgegenhaltungen:
- WO-A-99/11216
- DE-A- 10 027 480
- DE-A- 10 033 339
- DE-A- 10 058 363
- DE-A- 10 113 126

## Beschreibung

Die Erfindung bezieht sich zum einen auf eine Vorrichtung zur Erzeugung eines Schaumteppichs auf dem Boden.

In einer Reihe von hygienisch sensiblen Betrieben und Bereichen besteht immer die Forderung, das Eindringen von Krankheitserregern, nämlich pathogenen Keimen, wie Bakterien und Viren, in diese Betriebe und Bereiche zu verhindern. Als solche Bereiche und Betriebe sollen nur beispielhaft Landwirtschaftsbetriebe, Intensivtierhaltung, Tierumschlagspunkte, Tiermessen, Tierausstellungen, Schlachthöfe, Lebensmittel verarbeitende Betriebe, Staatsgrenzen, Sperrbezirke, Molkereien, Brauereien, insbesondere hinsichtlich der Herstellung und Verarbeitung von Maische, Zollstationen, Flughäfen, usw. genannt werden.

Da solche Keime insbesondere über die Sohlen von Schuhwerk sowie über Fahrzeugreifen in diese hygienisch sensiblen Bereiche eingeschleppt werden, ist es bekannt, in allen Zugangs- und Zufahrtsbereichen Desinfektionswannen und Desinfektionsmatten anzuordnen. Die in den hygienisch sensiblen Bereich hinein gehenden Personen betreten mit ihrem Schuhwerk diese Wannen und Matten, damit das Schuhwerk und insbesondere die Schuhsohle desinfiziert wird. Das entsprechende gilt für Fahrzeugreifen, die durch solche Wannen und über solche Matten fahren, um in den hygienisch sensiblen Bereich zu gelangen.

Bei dieser bekannten Vorrichtung kann eine mit Desinfektionslösung gefüllte Grube oder eine Wanne mit einer Vliesmatte oder einer Schaumstoffmatte vorgesehen sein, die mit der Desinfektionslösung getränkt ist. Sowohl mit der mit Desinfektionslösung gefüllten Grube als auch mit der Wanne mit eingelegter Matte wird nur ein unbefriedigendes Desinfektionsergebnis erreicht. Die Wirkung des Desinfektionsmittels lässt nämlich einerseits durch den ständigen Schmutzeintrag und andererseits infolge der Verdünnung mit Regenwasser schon nach relativ kurzer Zeit sehr stark nach. Um diesen Nachteil zu vermeiden, müsste die Desinfektionslösung in der Grube bzw. diese Lösung in der Wanne nach deren Reinigung mehrmals am Tag ausgetauscht werden, so dass dieses Desinfektionsverfahren umständlich, zeitaufwändig und wegen des erhöhten Chemikalien- und Wasserverbrauchs unwirtschaftlich ist. Ein weiterer Nachteil besteht darin, dass die bekannten Wannen mit eingelegten Vlies- oder Schaumstoffmatten nur wenig geeignet sind zum Desinfizieren von Fahrzeugreifen, da die Matten in kurzer Zeit durch das Gewicht sowie das häufig grobstollige Reifenprofil zerstört werden. Schließlich werden einige Wirkstoffe von Desinfektionslösungen, zum Beispiel Peressigsäure, Jodverbindungen, Chlorbleichlauge, usw., bereits bei Anwesenheit geringer Mengen von organischen Verschmutzungen zersetzt und damit wirkungslos. Solche Wirkstoffe von Desinfektionslösungen entfalten ihre Wirkung daher nur in frischen Lösungen.

Wird eine mit Desinfektionslösung gefüllte Grube verwendet, so werden die Laufflächen der Reifen und die Schuhsohlen in die Desinfektionslösung getaucht. Dabei ist aber nicht von außen ohne weiteres erkennbar, ob tatsächlich eine vollständige Benetzung der kritischen Flächen erfolgt ist. Schließlich kann der flüssige Desinfektionsmittelfilm von den Reifen bzw. von der Schuhsohle sehr schnell abtropfen, so dass die Wirkungsdauer des Desinfektionsmittels verkürzt ist. Der gleiche Nachteil stellt sich bei einer Vorrichtung ein, die mit einer Wanne mit eingelegter Matte arbeitet.

Bekannt ist außerdem ein Verfahren und eine Vorrichtung der Firma Ecolab Inc., USA, zur Desinfektion von Fahrzeugreifen sowie von Schuhwerk. Hier wird in Zugangsbereichen zu Lebensmittel verarbeitenden Betrieben eine Schleuse aufgebaut, bei der über jeweils eine seitlich angeordnete Düse ein desinfizierender Schaum auf das Schuhwerk einer Person bzw. auf die Reifen eines Fahrzeugs gesprüht wird. Der Boden der Schleuse besteht aus Fliesen, Beton, Kunststoff oder Metall und ist mit einem Abfluss versehen.

Vorteilhaft bei diesem Verfahren und dieser Vorrichtung ist es, dass immer ein frischer, unverbrauchter Schaum eingesetzt wird, welcher das Desinfektionsmittel enthält. Nachteilig dagegen ist es, dass der frische Schaum nur von den Seiten auf das Schuhwerk und die Reifen gelangt, so dass der besonders kritische Bereich, nämlich die Schuhsohle und die Lauffläche der Reifen, nicht immer mit Sicherheit erreicht werden. Schließlich kann der aus den Düsen austretende Schaumstrahl von Personen leicht übersprungen werden, welche die Befeuchtung ihres Schuhwerks vermeiden möchten. Zusätzlich ist es von Nachteil, dass der Schaum auch in das Schuhwerk gelangen kann und dass der auf dem Boden der Schleuse befindliche Schaum zu einer erhöhten Rutschgefahr führt. Ein schnelles Abfließen des verbrauchten Schaumes ist außerdem nicht gewährleistet und der verbleibende, organische Verschmutzungen enthaltende Schaum zersetzt schnell die mit dem frischen Schaum nachgelieferten, in Anwesenheit von organischen Bestandteilen instabilen Wirkstoffe.

Der Erfindung liegt daher die Aufgabe zugrunde, eine Vorrichtung und ein Verfahren zur Desinfektion von Fahrzeugreifen und von Schuhwerk zu entwickeln, wobei die Desinfektion ohne größeren Zeitaufwand durchgeführt werden kann, die Vorrichtung einfach und schnell installiert werden kann, einen nur geringen Wartungsaufwand erfordert, eine einfache und problemlose Benutzung ermöglicht, nur geringe Mengen an Chemikalien und Wasser verbraucht, auch für sich schnell zersetzende Chemikalien wie Peressigsäure, Jodverbindungen, Chlorbleichlauge, usw. geeignet ist und sowohl zum Desinfizieren von Schuhwerk als auch von Reifen schwerer Fahrzeuge eingesetzt werden kann.

Diese Aufgabe wird bei der eingangs genannten Vorrichtung erfindungsgemäß gelöst durch einen Bodenbelag mit einem Leitungssystem, welches mindestens einen Auslass, insbesondere mit einer oder mehreren Schaumdüsen, aufweist und zur Bildung eines Schaumes auf diesem Bodenbelag befähigt ist.

Erfindungsgemäß erfolgt die Desinfektion mit einem frisch erzeugten Schaum eines geeigneten Desinfektionsmittels mit einer für die spezifischen pathogenen Keime geeigneten Konzentration.

Da der Schaumteppich auf dem Bodenbelag gebildet wird und damit von unten an die kritischen Flächen, nämlich die Schuhsohlen und die Laufflächen der Reifen bzw. Räder der Fahrzeuge, gelangt, ist die Gefahr, dass der Schaum in das Schuhwerk des Personals eindringt, wesentlich verringert. Durch den auf dem Bodenbelag gebildeten Schaumteppich wird das Schuhwerk des Personals zwangsweise desinfiziert, soweit ein entsprechend großer, nicht vom Personal zu überspringender Bodenbelag bzw. Schaumteppich vorgesehen ist. Eine Möglichkeit für das Personal, eine Desinfektion des Schuhwerks zu vermeiden, ist nun ausgeschlossen. Da der Schaum mit dem Desinfektionswirkstoff flächendeckend und gleichmäßig auf die zu desinfizierenden Flächen aufgebracht wird, wird eine bessere Desinfektionswirkung erreicht.

Vorzugsweise verläuft das Leitungssystem im wesentlichen parallel zur Fläche des Bodenbelags und unterhalb und/oder insbesondere innerhalb des Bodenbelags.

Weiterhin wird vorgeschlagen, dass die Schaumdüsen unterhalb und/oder innerhalb des Bodenbelags angeordnet sind, damit einerseits eine Beschädigung der Schaumdüsen beim Betreten/Befahren des Bodenbelags durch Personen oder Fahrzeuge mit hoher Sicherheit vermieden wird und andererseits der desinfizierende frische Schaum mit hoher Sicherheit von unten auf die zu desinfizierenden Flächen gelangt.

Vorzugsweise weist das Leitungssystem mindestens einen Einlass auf, der mit einem Behälter für ein zur Schaumerzeugung geeignetes Mittel verbindbar ist.

Damit eine Verschmutzung des frischen Schaumes durch verunreinigten Restschaum von der vorhergehenden Desinfektion vermieden wird, ist es günstig, wenn der Bodenbelag Mittel zum schnellen Abfließen von Wasser, Schmutzwasser, verbrauchtem Schaum oder anderen frei fließenden Lösungen aufweist. Eine vorteilhafte Ausgestaltung der Erfindung besteht darin, dass die genannten Mittel zum schnellen Abfließen als Öffnungen im Bodenbelag ausgebildet sind und der Bodenbelag insbesondere eine gleichmäßige Gitterstruktur aufweist. Durch die Gitterstruktur des Bodenbelages ist die Gefahr, dass das Personal ausrutscht, weitgehend ausgeschlossen. Auch das schnelle Abfließen des verbrauchten Schaumes wird durch die Gitterstruktur des Bodenbelages begünstigt, so dass eine Verschmutzung des beim nachfolgenden Desinfektionsvorganges erzeugten Frischschaumes weiter verringert wird, denn mit dem verbrauchten Schaum fließt auch der eingetragene Schmutz von der Schuhsohle und von den Laufflächen der Reifen ab.

Wenn der Bodenbelag Mittel zum Halten eines Abstandes zu seiner Auflagefläche und dazu insbesondere auf seiner Unterseite Füße aufweist, können Wasser, Schmutzwasser, verbrauchter Schaum oder andere frei fließenden Lösungen noch schneller unterhalb des Bodenbelags abfließen. Für den Fall, dass der verbrauchte Schaum nicht in die Kanalisation gelangen darf, wird ein abschließbarer Abfluss vorgeschlagen.

Vorzugsweise hat der Bodenbelag eine Dicke von 0,5 bis 10 cm, insbesondere von 1 bis 8 cm.

Günstig ist es außerdem, wenn der Bodenbelag aus Metall und/oder insbesondere aus Kunststoff besteht. Ein Bodenbelag aus Kunststoff ist nämlich einerseits biegsam und passt sich einem unebenen Untergrund an. Er ist andererseits widerstandsfähig gegenüber Chemikalien.

Weiter wird vorgeschlagen, dass der Bodenbelag als eine Matte aus festem Material ausgebildet und geeignet ist, von einem oder mehreren Menschen oder Tieren betreten und/oder von Fahrzeugen befahren zu werden. Dabei ist unter dem Begriff "fest" zu verstehen, dass die geometrische Struktur der Matte weitgehend erhalten bleibt, wenn ein Fahrzeug über die Matte fährt und/oder ein Mensch oder Tier über die Matte geht.

Günstig ist es außerdem, wenn die Schaumdüsen von der Oberseite des Bodenbelags weg nach oben und/oder schräg nach oben gerichtet sind, um oberhalb des Bodenbelags den Schaumteppich zu erzeugen. Dabei ist es möglich, dass die einen Schaumdüsen senkrecht nach oben und die anderen Schaumdüsen schräg nach oben ausgerichtet sind.

Vorzugsweise sind die Schaumdüsen im Abstand von 10 bis 200 cm, insbesondere von 20 bis 150 cm, zueinander angeordnet.

Die Erfindung umfasst außerdem eine Hygiene-Schleuse im Zugangsbereich zu hygienisch sensiblen Gebieten, auf deren Bodenfläche sich eine erfindungsgemäße Vorrichtung befindet, die an einen oder mehreren Behältern angeschlossen ist, die für die Schaumerzeugung geeignete Mittel enthalten und deren Dosierung automatisch und/oder manuell gestartet wird, bevor und/oder sobald sich ein beweglicher Körper in die Hygiene-Schleuse begibt und/oder wenn sich der Körper innerhalb der Hygiene-Schleuse befindet. Der automatische Start erfolgt vorzugsweise durch Erkennung des Körpers über eine Lichtschranke, über Kontaktschalter oder beim Öffnen einer Tür oder einer Schranke. Auch eine manuelle Steuerung kann alternativ oder auch gleichzeitig mit der automatischen Steuerung vorgesehen sein.

Dabei sind vorzugsweise die Lichtschranken, Kontaktschalter oder andere entsprechende Sensoren räumlich so angeordnet, dass ein ausreichender Schaumteppich bereits beim Betreten der Hygiene-Schleuse vorliegt.

Von Vorteil ist es außerdem, wenn der Bodenbelag in oder oberhalb einer Vertiefung, insbesondere einer Wanne oder einer Grube, angeordnet ist, welche zum Auffangen von abgeflossenem Wasser, Schmutzwasser, verbrauchtem Schaum oder anderen frei fließenden Lösungen geeignet ist.

Schließlich ist es auch günstig, wenn die genannte Bodenfläche bzw. Vertiefung oder Wanne ein leichtes Gefälle aufweist, wodurch das Ablaufen von Wasser, Schmutzwasser, verbrauchtem Schaum oder anderen frei fließenden Lösungen in Richtung eines Abflusses ermöglicht wird, welcher vorzugsweise abschließbar ausgelegt ist, damit im Bedarfsfall, z. B. im Seuchenfall, keine verschmutzten und/oder Keime enthaltenden Schaumreste in die Kanalisation gelangen. Da das Ablaufen von verbrauchtem Schaum und darin dispergierten Schmutzpartikeln erleichtert wird, ist die Gefahr der Wiederverschmutzung reduziert und damit auch die Zehrung von weniger stabilen Wirkstoffen (Peressigsäure, Chlorbleichlauge, usw.).

Die Erfindung umfaßt außerdem ein Verfahren zur Behandlung von Fußbekleidung, wie Schuhen, Stiefeln und dergleichen, und/oder von Tierpfoten und/oder von Reifen und Rädern von Fahrzeugen mit Schaumreinigungsmitteln und/oder Schaumdesinfektionsmitteln im Zugangsbereich zu hygienisch sensiblen Gebieten, insbesondere zu seuchengefährdeten Gebieten oder aseptischen Bereichen im institutionellen oder industriellen Sektor, bei dem unter, über und/oder in dem Bodenbelag der erfindungsgemäßen Vorrichtung ein reinigender und/oder desinfizierender Schaum erzeugt wird und beim Betreten oder Befahren des genannten Zugangsbereichs mit der Fußbekleidung, den Tierpfoten und/oder den Reifen oder Rädern in Berührung gebracht wird.

Wie bereits oben ausgeführt, werden in dieser Anmeldung unter dem Begriff "hygienisch sensible Bereiche" Landwirtschaftsbetriebe, Intensivtierhaltung, Tierumschlagspunkte, Tiermessen und -ausstellungen, Schlachthöfe, Lebensmittel verarbeitende Betriebe, Staatsgrenzen, Sperrbezirke, usw. verstanden.

Die Erfindung umfasst weiterhin die Verwendung einer erfindungsgemäßen Vorrichtung und/oder einer erfindungsgemäßen Hygiene-Schleuse als ständige prophylaktische Maßnahme oder als Notfallausrüstung gegen die Ausbreitung von Seuchen oder Krankheitserregern in hygienisch sensiblen Betrieben und/oder Bereichen, z. B. in Landwirtschaftsbetrieben, Schlachthöfen, Lebensmittel verarbeitenden Betrieben und Molkereien sowie im Zugangsbereich zu aseptischen Abfüll- oder Produktionsstätten, ferner bei Intensivtierhaltung, Tierumschlagspunkten, Tiermessen und -ausstellungen, Schlachthöfen, Lebensmittel verarbeitenden Betrieben, Staatsgrenzen, Sperrbezirken, usw.

Schließlich betrifft die Erfindung auch die Verwendung einer erfindungsgemäßen Vorrichtung als Notfallausrüstung für Einsatzkräfte wie Feuerwehr, Polizei, Grenz- und Katastrophenschutz.

Im folgenden werden mehrere Ausführungsbeispiele der Erfindung anhand von Zeichnungen näher beschrieben. Es zeigen
- Figur 1: eine perspektivische Ansicht eines erfindungsgemäßen Bodenbelags,
- Figur 2: eine schematische Darstellung einer erfindungsgemäßen Hygiene-Schleuse in teilweise perspektivischer Ansicht vor dem Betreten durch eine Person bzw. vor dem Befahren durch ein Fahrzeug,
- Figur 3: die Hygiene-Schleuse nach Figur 2 kurz nach dem Betreten des Bodenbelags durch eine Person,
- Figur 4: die Hygiene-Schleuse nach Figur 2 kurz nach dem Einfahren eines Lastkraftwagens auf den Bodenbelag,
- Figur 5: eine alternative Ausführung der Hygiene-Schleuse nach den Figuren 2 bis 4 und
- Figur 6: die Hygiene-Schleuse nach Figur 5 kurz nach dem Einfahren eines Lastkraftwagens auf den Bodenbelag.

In allen Zeichnungen haben gleiche Bezugszeichen die gleiche Bedeutung und werden daher gegebenenfalls nur einmal erläutert.

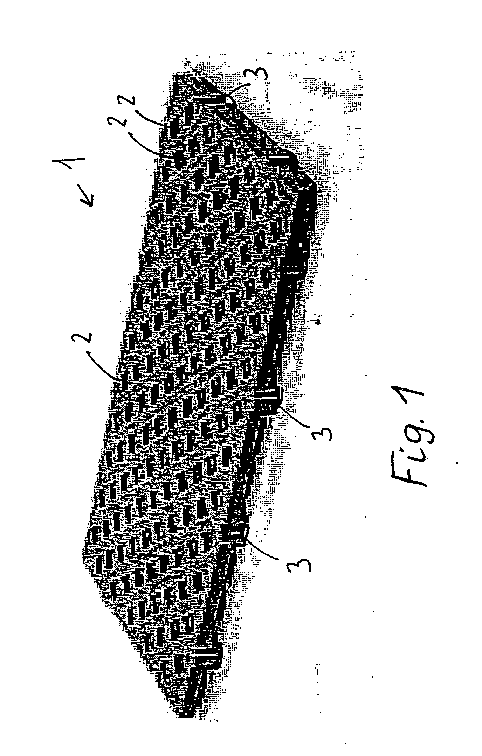

Das wesentliche Element der erfindungsgemäßen Vorrichtung bildet der Bodenbelag, welcher im Ausführungsbeispiel als eine Matte 1 in Figur 1 dargestellt ist. Die Matte 1 ist mit Schaumdüsen, welche in Figur 1 nicht eingezeichnet sind, versehen. An Stelle von Schaumdüsen kann auch ein Rohr oder ein Schlauch mit entsprechenden Bohrungen vorgesehen sein.

Eine bestimmte Mindestgröße für die Matte 1 ist nicht notwendig. Bei der Aufstellung der Matte in der Praxis sollte jedoch eine Mindestfläche des Bodens mit einer oder mehreren Matten bedeckt werden, wobei diese Mindestfläche vom Einsatzgebiet abhängt. Falls nur Schuhwerk desinfiziert werden soll, muss die Matte mindestens so groß sein, dass der Schuh oder Stiefel bequem auf die Matte passt. Sollen Fahrzeugreifen desinfiziert werden, so sollte die Matte mindestens so groß sein, dass der in der Praxis vorkommende größte und breiteste Reifen beim Durchfahren der entsprechenden Schleuse in vollem Umfang mit der Oberfläche der Matte in Berührung kommt.

In der Praxis sind die Mattenflächen jedoch in der Regel erheblich größer als die genannte Mindestgröße, damit die Benetzung des Schuhwerks bzw. der Reifen mit dem Desinfektionsmittel-Schaum mit Sicherheit erfolgt und außerdem die Einwirkzeit verlängert ist.

Das Leitungssystem mit den Schaumdüsen kann fest montiert oder modular anbaufähig sein. Dabei können mehrere Matten mit ihren Schaumrohren miteinander verbunden werden, um einen Schaumteppich mit einer größeren Fläche zu ermöglichen.

Der Abstand der Schaumdüsen bzw. der Bohrungen im Schaumrohr oder Schlauch hängt von dem an Ort und Stelle benötigten Schaumvolumen ab. Dieses wiederum hängt von der Größe der mit einer oder mehreren Matten ausgestatteten Fläche, von der Nutzungshäufigkeit, von der Menge des eingetragenen Schmutzes und vom Wirkstoffverbrauch ab. Der Abstand der Düsen oder der Bohrungen des Schaumrohrs/Schlauches kann im Bereich von 10 cm bis 200 cm, vorzugsweise im Bereich von 20 cm bis 150 cm liegen.

Die Anordnung der Matte 1 bzw. mehrerer miteinander verbundener oder nicht verbundener Matten in einer Wanne oder Grube sollte vorzugsweise mit einem zu einem Abfluss hin gerichteten Gefälle vorgenommen werden, wobei der Abfluss vorzugsweise verschließbar ist. Auf diese Weise kann der verbrauchte flüssige und den Schmutz tragende Schaum leichter abfließen. Dabei kann die Wanne aus Metall oder Kunststoff bestehen. Vorzugsweise hat die Matte 1 eine relativ große Dicke, nämlich von 0,5 bis 10 cm, insbesondere von 1 bis 8 cm. Zum Abfließen des verbrauchten Schaumes ist eine Vielzahl von durchgehenden Öffnungen 2 in der Matte vorgesehen, die regelmäßig angeordnet sind und eine Gitterstruktur bilden. Zusätzlich sorgen Füße 3 an der Unterseite der Matte 1 für ein leichtes Abfließen des verbrauchten Schaumes, da die Unterseite der Matte im Abstand vom Boden angeordnet ist.

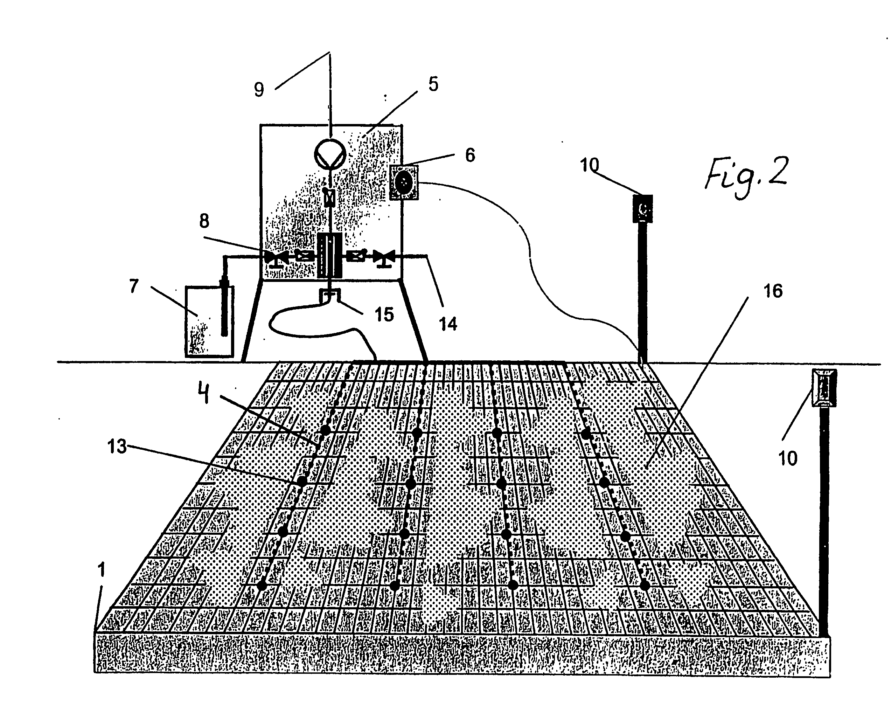

Der Aufbau einer Hygiene-Schleuse mit einer Matte nach Figur 1 ist schematisch in Figur 2 dargestellt. Innerhalb des wasserdurchlässigen Bodenbelags 1 ist ein Leitungssystem 4 mit Schaumdüsen 13 angeordnet, welche nach oben hin ausgerichtet sind. Die Schaumdüsen 13 des Leitungssystems 4 können übliche Düsen sein. Alternativ kann auch ein Rohr oder ein Schlauch mit entsprechend geeigneten Bohrungen vorgesehen sein. Die Düsen und die Bohrungen sind so angebracht, dass eine ausreichende Menge an Schaum auf der Oberfläche der Matte 1 aufliegt, wenn das Schaumerzeugungsgerät 5 arbeitet.

Das Leitungssystem 4 ist mit seinem Einlass an ein schematisch dargestelltes Schaumerzeugungsgerät 5 angeschlossen, welches von einer Steuereinheit 6 betätigt werden kann. Zum Erzeugen des desinfizierenden Schaumes ist das Schaumerzeugungsgerät 5 über eine Desinfektionsmittelleitung 8 an einen Behälter 7 für das Desinfektionsmittel angeschlossen, aus dem dieses Mittel angesaugt wird, beispielsweise mittels eines Venturi-Rohrs. Das zur Erzeugung des Schaumes erforderliche Wasser wird über eine Wasserzulaufleitung 9 dem Schaumerzeugungsgerät 5 zugeführt. Ferner ist noch eine in Figur 2 nicht dargestellte Einrichtung zur Erzeugung von für die Erzeugung des Schaumes benötigter Druckluft im Schaumerzeugungsgerät 5 vorgesehen. Andererseits kann die Druckluft auch von einer externen Druckluftquelle über die Anschlussleitung 14 in das Schaumerzeugungsgerät 5 eingespeist werden.

Die Steuereinheit 6 erhält ihre Eingangssignale von einer Lichtschranke 10, die in an sich bekannter Weise aus einer im Abstand von einer Lichtquelle angeordneten Fotozelle besteht.

Als Schaumerzeugungsgerät kann ein an sich bekanntes, kommerzielles Gerät, z. B. das Gerät "S 6000" der Fa. ALTO eingesetzt werden. Vorzugsweise wird das Schaumerzeugungsgerät über eine Schnellkupplung 15 mit dem Leitungssystem 4 verbunden. Schließlich sind in Figur 2 Reste des vom vorhergehenden Desinfektionsvorgang stammenden Schaumteppichs 16 zu erkennen.

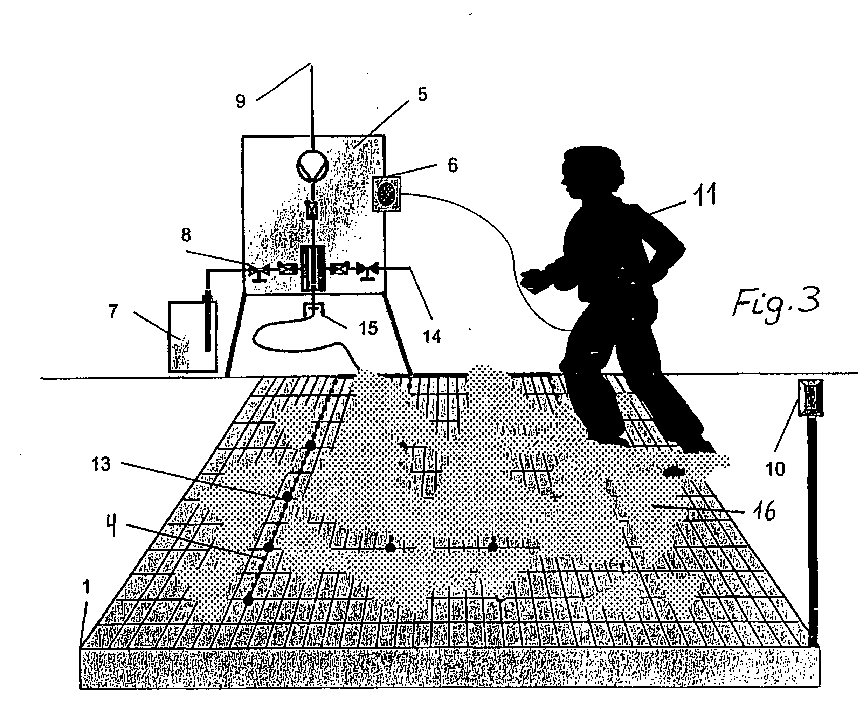

In Figur 3 ist die Hygiene-Schleuse nach Figur 2 im Zustand dargestellt, wenn eine Person 11 gerade die Schleuse betreten hat. Nach dem Unterbrechen des Lichtstrahls der Lichtschranke 10 gibt die Steuereinheit 6 ein Signal an das Schaumerzeugungsgerät, so dass dieses betätigt und Schaum über die Schaumdüsen des Leitungssystems 4 an die Oberfläche der Matte 1 abgegeben wird. Praktisch das gesamte Schuhwerk, zumindest aber dessen unteren Teile, insbesondere die Sohlenflächen, sind vollständig und vollflächig dick mit dem desinfizierenden Schaum bedeckt.

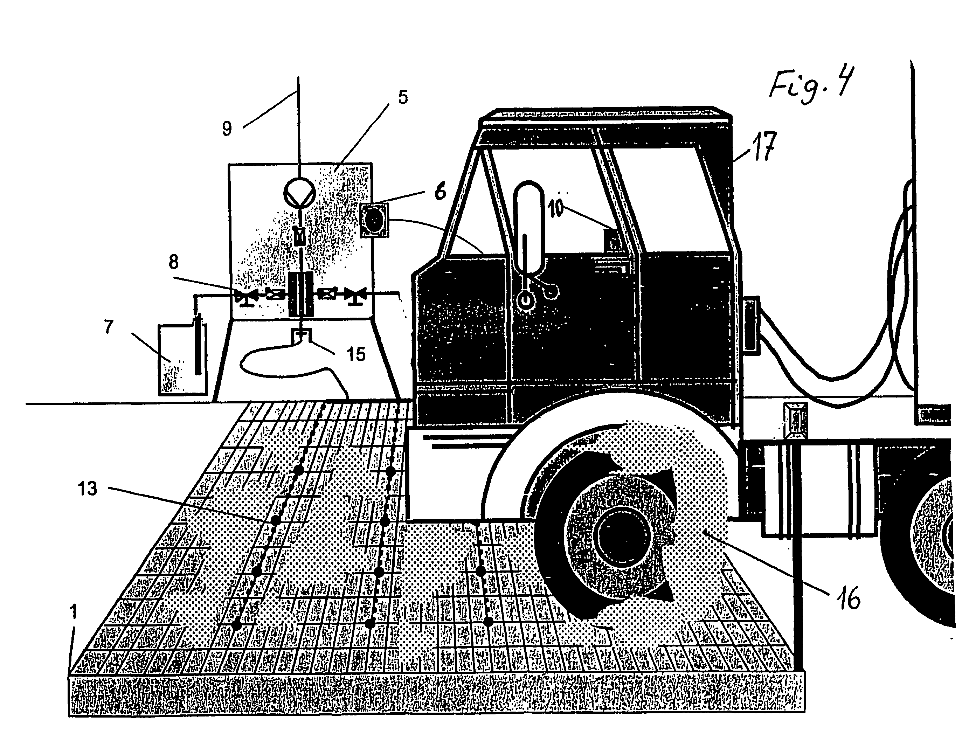

In entsprechender Weise wie in Figur 3 zeigt Figur 4 die Hygiene-Schleuse nach Figur 2 während des Befahrens durch einen Lastkraftwagen 17. Erkennbar sind die kritischen Stellen, nämlich die Reifen im Bereich des Laufprofils, vollständig und dick mit dem desinfizierenden Schaum bedeckt.

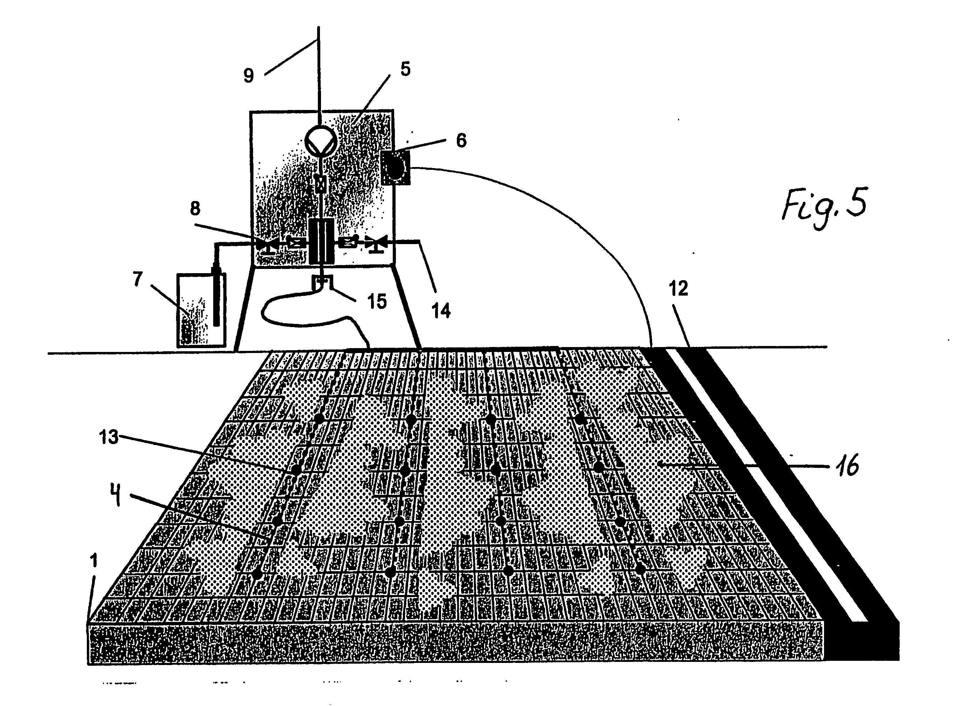

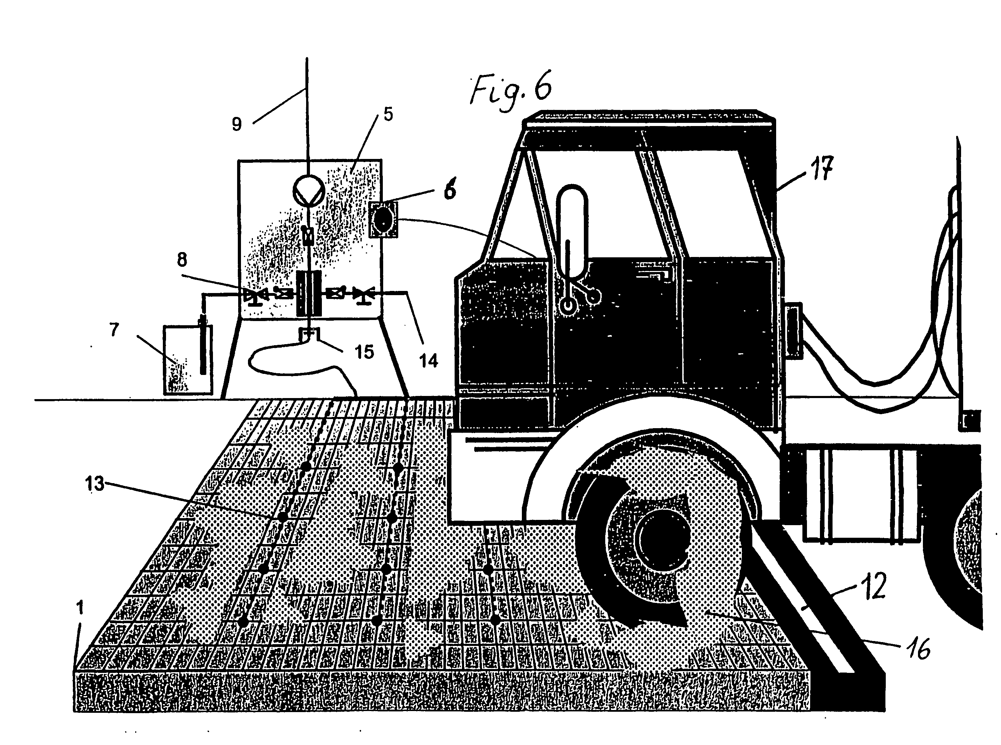

Die Aktivierung der Schaumdüsen 13 kann auch manuell oder ebenfalls automatisch über einen Kontaktschalter 12 (Figuren 5 und 6) am Boden im Eingangsbereich der Hygiene-Schleuse erfolgen. Es kann auch vorgesehen sein, dass das Schaumerzeugungsgerät beim Öffnen einer Tür oder einer Schranke aktiviert wird, wobei der Vorteil in der Unterscheidung von unreinen, so genannten schwarzen, und reinen, so genannten weißen, Bereichen liegt, wobei diese Bereiche üblicherweise ohnehin schon durch eine bereits vorhandene Tür oder Schranke getrennt sind. Diese bereits vorhandene Tür oder Schranke kann dann zur Aktivierung des Schaumerzeugungsgeräts 5 über die Steuereinheit 6 eingesetzt werden.

Wenn Personen die Hygiene-Schleuse passieren oder Fahrzeuge diese Schleuse durchfahren, werden die Schuhsohlen bzw. Reifen mit frischem Desinfektionsschaum bedeckt. Der Schaum fließt in alle Ecken und Kanten, d. h. auch in tiefe Rillen des Schuh- oder Reifenprofils und bleibt dort für längere Zeit haften, so dass die Einwirkzeit des Desinfektionsmittels verlängert ist. Der Schaum ist außerdem sichtbar, so dass auf einfache Weise kontrolliert werden kann, ob sämtliche zu desinfizierenden Flächen vollständig mit dem Desinfektionsmittel behandelt werden.

Mit dem erfindungsgemäßen Verfahren können auch Wirkstoffe eingesetzt werden, die in der Lösung in Anwesenheit von Verschmutzungen instabil sind, da erfindungsgemäß der Schaum immer frisch hergestellt wird. Der verbrauchte Schaum zerfällt leicht und fließt durch die Öffnungen 2 der Matte 1 ab. Durch die automatische oder manuelle Aktivierung des Schaumerzeugungsgeräts braucht man keine frische Lösung anzusetzen, so dass hier eine Einsparung an Zeit, Wasser und Chemikalien erreicht wird.

Die erfindungsgemäße Vorrichtung und Hygiene-Schleuse kann im Bedarfsfall einfach und schnell installiert werden und insbesondere als Maßnahme gegen die Ausbreitung von Seuchen an hygienisch sensiblen Bereichen aufgestellt werden, z. B. an Staats-, Landes- oder Orts-Grenzen, in Seuchen-Sperrbezirken, in Betrieben der Lebensmittel-, Milch- und Getränke-Industrie sowie in Landwirtschaftsbetrieben, usw. Auch als präventive Maßnahme gegen eine befürchtete Ausbreitung von Seuchen und Krankheiten und zur Vermeidung einer Kreuzkontamination, nämlich der Verschleppung pathogener Keime von einem Bereich in den nächsten, sind die erfindungsgemäße Vorrichtung und die Hygiene-Schleuse sowie das erfindungsgemäße Verfahren besonders gut geeignet. In Betrieben der Lebensmittel- und Getränke-Industrie kann die Hygiene-Schleuse als Barriere gegen das Einschleppen von Krankheitserregern eingesetzt werden. Darüber hinaus kann die Vorrichtung und Hygiene-Schleuse als Notfall-Ausrüstung bei einem Seuchenfall für Feuerwehren, Polizei, Grenz- und Katastrophenschutz verwendet werden.

### Beispiele

Es wurden zwei Personalschleusen eingerichtet und die Bedingungen in der Praxis, z. B. in einem Landwirtschaftsbetrieb, simuliert.

### Versuch 1: Erfindungsgemäße Hygiene-Schleuse

Eine Matte mit eingebauten Schaumdüsen im Abstand von 30 cm wurde in einem mit einem Abfluss versehenen Korridor angeordnet. Die Schaumdüsen wurden über eine Schnellkupplung an ein Schaumerzeugungsgerät des Typs "ALTO S 2000" der Fa. ALTO angeschlossen. Das Schaumerzeugungsgerät wurde manuell bei jedem Passieren einer Person aktiviert, wobei dann das Peressigsäure und Tenside enthaltende Desinfektionsmittel "P3-topactive DES" (Produkt der Fa. Ecolab GmbH & Co. OHG) in einer Konzentration von 2 Gew.-% eingeschäumt wurde.

### Versuch 2: Vergleichsbeispiel

Bei einer aus dem Stand der Technik bekannten Hygiene-Schleuse zur Desinfektion von Schuhwerk wurde eine Schaumstoffmatte in eine Wanne eingelegt und die Matte mit einer Lösung von "P3-topactive DES" in einer Konzentration von 2 Gew.-% übergossen.

Beide Hygiene-Schleusen wurden von jeweils 100 Personen mit einer für den Landwirt üblichen Verschmutzung des Schuhwerks passiert. Jede Stunde passierten jeweils 10 Personen die Schleusen. Nach jeder 10. Passage wurde die Aktivität des Desinfektionswirkstoffes Peressigsäure an der Oberfläche der Matte als Maß für die Wirksamkeit der Hygiene-Schleuse getestet. Die Ergebnisse sind in Tabelle 1 darstellt:

**Tabelle 1:**

| Aktivität des Desinfektionswirkstoffes in % an der Oberfläche der Matte, wobei die Aktivität zu Beginn der Versuche jeweils auf 100 % gesetzt worden ist. | | | | | | | | | | | |
|---|---|---|---|---|---|---|---|---|---|---|---|
| | Personenpassagen | | | | | | | | | | |
| | 0 | 10 | 20 | 30 | 40 | 50 | 60 | 70 | 80 | 90 | 100 |
| Versuch 1 Aktivität | 10 | 95 | 97 | 95 | 93 | 88 | 95 | 96 | 92 | 93 | 91 |
| Versuch 2 Aktivität | 10 | 98 | 92 | 87 | 78 | 61 | 43 | 30 | 26 | 28 | 22 |

Die Ergebnisse zeigen eindeutig, dass bei der herkömmlichen Methode (Versuch 2) mit zunehmender Verschmutzung und Einsatzzeit die Wirksamkeit des Desinfektionsmittels abnimmt. Im erfindungsgemäßen Verfahren dagegen ist die Wirksamkeit innerhalb der üblichen Schwankungen über die gesamte Einsatzzeit konstant.

### Bezugszeichenliste

- 1: Matte, Bodenbelag
- 2: Öffnung
- 3: Fuß
- 4: Leitungssystem
- 5: Schaumerzeugungsgerät
- 6: Steuereinheit
- 7: Behälter für Desinfektionsmittel
- 8: Desinfektionsmittelleitung
- 9: Wasserzulaufleitung
- 10: Lichtschranke
- 11: Person
- 12: Kontaktschalter
- 13: Schaumdüsen
- 14: Anschlussleitung für Druckluftquelle
- 15: Schnellkupplung
- 16: Schaumteppich
- 17: Lastkraftwagen

## Patentansprüche

1. Vorrichtung zur Erzeugung eines Schaumteppichs auf dem Boden,
**gekennzeichnet durch**
einen Bodenbelag (1) mit einem Leitungssystem (4), welches mindestens einen Auslass, mit einer oder mehreren Schaumdüsen (13) aufweist und zur Bildung eines Schaumes auf diesem Bodenbelag (1) befähigt ist und wobei die Schaumdüsen (13) unterhalb und/oder innerhalb des Bodenbelags (1) angeordnet sind.

2. Vorrichtung nach Anspruch 1,
**dadurch gekennzeichnet,**
**dass** das Leitungssystem (4) im Wesentlichen parallel zur Fläche des Bodenbelags (1) und unterhalb und/oder innerhalb des Bodenbelags (1) verläuft.

3. Vorrichtung nach einem der vorhergehenden Ansprüche,
**dadurch gekennzeichnet,**
**dass** das Leitungssystem (4) mindestens einen Einlass aufweist, der mit einem Behälter (7) für ein zur Schaumerzeugung geeignetes Mittel verbindbar ist.

4. Vorrichtung nach einem der vorhergehenden Ansprüche
**dadurch gekennzeichnet,**
**dass** der Bodenbelag (1) Mittel zum schnellen Abfließen von Wasser, Schmutzwasser, verbrauchtem Schaum oder anderen frei fließenden Lösungen aufweist.

5. Vorrichtung nach Anspruch 4,
**dadurch gekennzeichnet,**
**dass** die genannten Mittel zum schnellen Abfließen als Öffnungen (2) im Bodenbelag (1) ausgebildet sind und der Bodenbelag (1) eine gleichmäßige Gitterstruktur aufweist.

6. Vorrichtung nach einem der vorhergehenden Ansprüche,
**dadurch gekennzeichnet,**
**dass** der Bodenbelag (1) Mittel zum Halten eines Abstandes zu seiner Auflagefläche und dazu insbesondere auf seiner Unterseite Füße (3) aufweist.

7. Vorrichtung nach einem der vorhergehenden Ansprüche,
**dadurch gekennzeichnet,**
**dass** der Bodenbelag (1) eine Dicke von 0,5 bis 10 cm, insbesondere von 1 bis 8 cm hat.

8. Vorrichtung nach einem der vorhergehenden Ansprüche,
**dadurch gekennzeichnet,**
**dass** der Bodenbelag (1) aus Metall und/oder aus Kunststoff besteht.

9. Vorrichtung nach einem der vorhergehenden Ansprüche,
**dadurch gekennzeichnet,**
**dass** der Bodenbelag als eine Matte (1) aus festem Material ausgebildet und geeignet ist, von einem oder mehreren Menschen oder Tieren betreten und/oder von Fahrzeugen befahren zu werden.

10. Vorrichtung nach einem der vorhergehenden Ansprüche,
**dadurch gekennzeichnet,**
**dass** die Schaumdüsen (13) von der Oberseite des Bodenbelags (1) weg nach oben und/oder schräg nach oben gerichtet sind.

11. Vorrichtung nach einem der vorhergehenden Ansprüche,
**dadurch gekennzeichnet,**
**dass** die Schaumdüsen (13) im Abstand von 10 bis 200 cm, insbesondere von 20 bis 150 cm, zueinander angeordnet sind.

12. Hygiene-Schleuse im Zugangsbereich zu hygienisch sensiblen Gebieten, auf deren Bodenfläche sich eine Vorrichtung nach einem der Ansprüche 1 bis 11 befindet, die an einen oder mehreren Behältern (7) angeschlossen ist, die für die Schaumerzeugung geeignete Mittel enthalten und deren Dosierung automatisch und/oder manuell gestartet wird, bevor und/oder sobald sich ein beweglicher Körper (11) in die Hygiene-Schleuse begibt und/oder wenn sich der Körper (11) innerhalb der Hygiene-Schleuse befindet.

13. Hygiene-Schleuse nach Anspruch 12,
**dadurch gekennzeichnet,**
**dass** der Bodenbelag (1) in oder oberhalb einer Vertiefung, insbesondere einer Wanne oder einer Grube angeordnet ist, welche zum Auffangen von abgeflossenem Wasser, Schmutzwasser, verbrauchtem Schaum oder anderen frei fließenden Lösungen geeignet ist.

14. Hygiene-Schleuse nach Anspruch 12 oder 13,
**dadurch gekennzeichnet,**
**dass** die genannte Bodenfläche bzw. Vertiefung oder Wanne ein leichtes Gefälle aufweist.

15. Verfahren zur Behandlung von Fußbekleidung, wie Schuhen, Stiefeln und dergleichen und/oder von Tierpfoten und/oder von Reifen und Rädern von Fahrzeugen mit Schaumreinigungsmitteln und/oder Schaumdesinfektionsmittein im Zugangsbereich zu hygienisch sensiblen Gebieten, insbesondere zu seuchengefährdeten Gebieten oder aseptischen Bereichen im institutionellen oder industriellen Sektor, bei dem unter, über und/oder in dem Bodenbelag (1) der Vorrichtung gemäß einem der Ansprüche1 bis 11 ein reinigender und/oder desinfizierender Schaum erzeugt wird und beim Betreten oder Befahren des genannten Zugangsbereichs mit der Fußbekleidung, den Tierpfoten und/oder den Reifen oder Rädern in Berührung gebracht wird.

16. Verwendung einer Vorrichtung gemäß einem der Ansprüche 1 bis 11 und/oder einer Hygiene-Schleuse gemäß einem der Ansprüche 12 bis 14 als ständige prophylaktische Maßnahme oder als Notfallausrüstung gegen die Ausbreitung von Seuchen oder Krankheitserregern in hygienisch sensiblen Betrieben und /oder Bereichen.

17. Verwendung einer Vorrichtung gemäß einem der Ansprüche 1 bis 11 als Notfallausrüstung für Einsatzkräfte wie Feuerwehr, Polizei, Grenz- und Katastrophenschutz.

## Claims

1. A device for the production of a foam carpet on the floor,
**characterized by**
a floor cover (1) with a conduit system (4), which conduit system has at least one outlet, with one or more foam nozzles (13), and is capable of forming a foam on said floor cover (1), said foam nozzles (13) being arranged beneath and/or within said floor cover (1).

2. The device according to claim 1,
**characterized in that**
the conduit system (4) essentially extends parallel to the surface of the floor cover (1) and beneath and/or within the floor cover (1).

3. The device according to any of the preceding claims,
**characterized in that**
the conduit system (4) has at least one inlet which can be connected to a container (7) for an agent suitable for foam production.

4. The device according to any of the preceding claims,
**characterized in that**
the floor cover (1) has means for rapid discharge of water, dirty water, used foam or other free-flowing solutions.

5. The device according to claim 4,
**characterized in that**
said means for rapid discharge are designed as apertures (2) in the floor cover (1), and that the floor cover (1) has a regular grid structure.

6. The device according to any of the preceding claims,
**characterized in that**
the floor cover (1) has means for keeping a distance to the supporting surface thereof and, in addition, sockets (3) particularly on the underside thereof.

7. The device according to any of the preceding claims,
**characterized in that**
the floor cover (1) has a thickness of from 0.5 to 10 cm, particularly from 1 to 8 cm.

8. The device according to any of the preceding claims,
**characterized in that**
the floor cover (1) is made of metal and/or plastic.

9. The device according to any of the preceding claims,
**characterized in that**
the floor cover (1) is designed in the form of a mat of solid material and suitable for allowing walking of one or more persons or animals and/or driving of vehicles thereon.

10. The device according to any of the preceding claims,
**characterized in that**
the foam nozzles (13) are directed upwardly away from the upper side of the floor cover (1) and/or slantedly upwardly.

11. The device according to any of the preceding claims,
**characterized in that**
the foam nozzles (13) are arranged at a distance of from 10 to 200 cm, particularly from 20 to 150 cm, with respect to each other.

12. A hygiene lock in the area of access to hygienically sensitive areas, having a device according to any of claims 1 to 11 arranged on the bottom surface thereof, which is connected to one or more containers (7) containing agents suitable for foam production, the metering of which is initiated automatically and/or manually prior to and/or as soon as a moving body (11) enters the hygiene lock and/or when said body (11) is situated inside the hygiene lock.

13. The hygiene lock according to claim 12,
**characterized in that**
the floor cover (1) is arranged in or above a depression, especially a basin or a pit, which is suitable for receiving discharged water, dirty water, used foam or other free-flowing solutions.

14. The hygiene lock according to claim 12 or 13,
**characterized in that**
said bottom surface or depression or basin is slightly sloped.

15. A method for the treatment of footwear, such as shoes, boots and the like, and/or animal paws and/or tires and wheels of vehicles with foam cleaning agents and/or foam disinfectants in the area of access to hygienically sensitive areas, particularly areas involving danger of an epidemic or aseptic areas in the institutional or industrial sector, in which method a cleaning and/or disinfecting foam is produced beneath, above and/or inside the floor cover (1) of the device according to any of claims 1 to 11 and contacted with said footwear, animal paws and/or tires or wheels when walking or driving on the above-mentioned area of access.

16. Use of a device according to any of claims 1 to 11 and/or a hygiene according to any of claims 12 to 14 as a permanent prophylactic measure or as an emergency equipment used to control spreading of epidemics or pathogens in hygienically sensitive businesses and/or areas.

17. Use of a device according to any of claims 1 to 11 as emergency equipment for task forces such as fire brigade, police, border protection and emergency services.

## Revendications

1. Dispositif de production d'un tapis mousse sur le sol, **caractérisé par** un revêtement de sol (1) avec un système de conduites (4), lequel comprend au moins une sortie, avec un ou plusieurs injecteur(s) de mousse (13) et qui est capable de former une mousse sur ce revêtement de sol (1), et les injecteurs de mousse (13) étant disposés au-dessous et/ou à l'intérieur du revêtement de sol (1).

2. Dispositif selon la revendication 1, **caractérisé en ce que** le système de conduites (4) s'étend sensiblement parallèlement à la surface du revêtement de sol (1) et au-dessous et/ou à l'intérieur du revêtement de sol (1).

3. Dispositif selon l'une quelconque des revendications précédentes, **caractérisé en ce que** le système de conduites (4) comprend au moins une entrée qui peut être reliée à un récipient (7) pour des moyens convenant à la production de mousse.

4. Dispositif selon l'une quelconque des revendications précédentes, **caractérisé en ce que** le revêtement de sol (1) comprend des moyens destinés à laisser s'écouler rapidement l'eau, l'eau sale, la mousse usagée ou d'autres solutions circulant librement.

5. Dispositif selon la revendication 4, **caractérisé en ce que** les moyens cités destinés à l'écoulement rapide sont conçus comme des orifices (2) dans le revêtement de sol et le revêtement de sol (1) comprend une structure grillagée régulière.

6. Dispositif selon l'une quelconque des revendications précédentes, **caractérisé en ce que** le revêtement de sol (1) comprend des moyens destinés à maintenir un écart par rapport à sa surface d'appui et notamment à cet effet des pieds (3) sur sa face inférieure.

7. Dispositif selon l'une quelconque des revendications précédentes, **caractérisé en ce que** le revêtement de sol (1) présente une épaisseur de 0,5 à 10 cm, notamment de 1 à 8 cm.

8. Dispositif selon l'une quelconque des revendications précédentes, **caractérisé en ce que** le revêtement de sol (1) est constitué de métal et/ou de matière plastique.

9. Dispositif selon l'une quelconque des revendications précédentes, **caractérisé en ce que** le revêtement de sol est conçu comme un tapis (1) en matériau solide et convient pour que plusieurs personnes ou animaux marchent dessus et/ou que des véhicules roulent dessus.

10. Dispositif selon l'une quelconque des revendications précédentes, **caractérisé en ce que** les injecteurs de mousse (13) sont orientés vers le haut à distance de la face supérieure du revêtement de sol (1) et/ou vers le haut de façon inclinée.

11. Dispositif selon l'une quelconque des revendications précédentes, **caractérisé en ce que** les injecteurs de mousse (13) sont disposés les uns par rapport aux autres selon une distance de 10 à 200 cm, notamment de 20 à 150 cm.

12. Sas sanitaire pour une zone d'accès à des domaines sensibles d'un point de vue hygiénique, sur la surface inférieure duquel se situe un dispositif selon l'une quelconque des revendications 1 à 11, qui est raccordé à un ou plusieurs récipient(s) (7), qui contient(contiennent) des moyens appropriés pour la production de mousse et dont le dosage démarre automatiquement et/ou manuellement, avant et/ou dès qu'un corps mobile (11) pénètre en marchant dans le sas sanitaire et/ou lorsque le corps (11) se situe dans le sas sanitaire.

13. Sas sanitaire selon la revendication 12, **caractérisé en ce que** le revêtement de sol (1) est disposé dans un renfoncement ou au-dessus de celui-ci, notamment une cuvette ou une fosse, laquelle convient pour réceptionner l'eau, l'eau sale, la mousse usagée s'écoulant ou d'autres solutions circulant librement.

14. Sas sanitaire selon la revendication 12 ou 13, **caractérisé en ce que** la surface inférieure citée et/ou le renfoncement ou la cuvette présentent une légère déclivité.

15. Procédé destiné à traiter les chaussures, comme les bottes et similaires et/ou les pattes d'animaux et/ou les pneus et les roues de véhicules avec des moyens de nettoyage par la mousse et/ou des moyens de désinfection par la mousse dans la zone d'accès à des domaines sensibles d'un point de vue hygiénique, notamment des domaines présentant un risque épidémique ou des zones aseptiques dans le secteur institutionnel ou industriel, dans lequel, au-dessous, au-dessus et/ou à l'intérieur du revêtement de sol (1) du dispositif selon l'une quelconque des revendications 1 à 11, une mousse nettoyante et/ou désinfectante est produite et, lorsqu'on pénètre à pied ou en véhicule dans la zone d'accès citée, ladite mousse est mise en contact avec les chaussures, les pattes d'animaux et/ou les pneus ou les roues.

16. Utilisation d'un dispositif selon l'une quelconque des revendications 1 à 11 et/ou d'un sas sanitaire selon l'une quelconque des revendications 12 à 14 comme mesure prophylactique permanente ou comme équipement d'urgence pour lutter contre la propagation d'épidémies ou d'éléments pathogènes dans des domaines et/ou des zones sensibles d'un point de vue hygiénique.

17. Utilisation d'un dispositif selon l'une quelconque des revendications 1 à 11 comme équipement d'urgence pour les forces d'intervention comme les pompiers, la police, la protection des frontières et la gestion des catastrophes.
